# EUROPEAN PATENT SPECIFICATION

(11) **EP 2 109 452 B1**
(45) Date of publication and mention of the grant of the patent: **31.07.2013**
(21) Application number: 08709024.7
(22) Date of filing: 15.02.2008
(51) Int. Cl.: A61K 35/74, A61K 39/395, A61K 45/06, A61P 35/00

(54) **USE OF A NEUTROPHILIC GRANULOCYTE DIMINISHING OR INACTIVATING AGENT IN COMBINATION WITH BACTERIALLY-MEDIATED TUMOR TREATMENT**
VERWENDUNG EINES NEUTROPHILE GRANULOZYTENABSCHWÄCHUNGS- ODER -DEAKTIVIERUNGSMITTELS IN KOMBINATION MIT EINER BAKTERIELL VERMITTELTEN TUMORBEHANDLUNG
UTILISATION D'UN AGENT RÉDUISANT OU INACTIVANT DES GRANULOCYTES NEUTROPHILES, EN ASSOCIATION À UN TRAITEMENT DES TUMEURS À MÉDIATION BACTÉRIENNE

(30) Priority: 15.02.2007 EP 07102516
(43) Date of publication of application: 21.10.2009
(73) Proprietor: Helmholtz-Zentrum für Infektionsforschung GmbH, 38124 Braunschweig (DE)
(72) Inventor: WESTPHAL, Kathrin, 38106 Braunschweig (DE); LÖSSNER, Holger, 63225 Langen (DE); LESCHNER, Sara, 38114 Braunschweig (DE); WEISS, Siegfried, 38118 Braunschweig (DE)
(74) Representative: Taruttis, Stefan Georg
(86) International application number: PCT/EP2008/051839
(87) International publication number: WO 2008/099001

(56) References cited:
- EP-A- 1 655 370
- WO-A-01/24637
- "Final Program" 37TH ANNUAL MEETING OF THE GERMAN SOCIETY FOR IMMUNOLOGY, [Online] 5 September 2007 (2007-09-05), pages 1-225, XP002480881 Retrieved from the Internet: URL:http://www.dgfi.dbkr.de/fileadmin/pdf/ heidelberg_program.pdf> [retrieved on 2008-05-19] & WESTPHAL KATHRIN ET AL: "Containment of tumor colonizing bacteria by host neutrophols" 37TH ANNUAL MEETING OF THE GERMAN SOCEITY FOR IMMUNOLOGY, ABSTRACTS, [Online] 5 September 2007 (2007-09-05), pages 1-757, Heidelberg Retrieved from the Internet: URL:http://www.dgfi.dbkr.de/fileadmin/pdf/ dgfi_abstracts_fall07.pdf> [retrieved on 2008-05-19]
- WESTPHAL KATHRIN ET AL: "Containment of tumor-colonizing bacteria by host neutrophils." CANCER RESEARCH 15 APR 2008, vol. 68, no. 8, 15 April 2008 (2008-04-15), pages 2952-2960, XP002480882 ISSN: 1538-7445
- PAWELEK J M ET AL: "Bacteria as tumour-targeting vectors" LANCET ONCOLOGY, LANCET PUBLISHING GROUP, LONDON, GB, vol. 4, no. 9, 1 September 2003 (2003-09-01), pages 548-556, XP004809880 ISSN: 1470-2045
- NOUGAYRÈDE JEAN-PHILIPPE ET AL: "Escherichia coli induces DNA double-strand breaks in eukaryotic cells" SCIENCE, WASHINGTON, DC, vol. 313, no. 5788, 11 August 2006 (2006-08-11), pages 848-851, XP002423942 ISSN: 0036-8075
- GENESTIER ANNE-LAURE ET AL: "Staphylococcus aureus Panton-Valentine leukocidin directly targets mitochondria and induces Bax-independent apoptosis of human neutrophils" JOURNAL OF CLINICAL INVESTIGATION, NEW YORK, NY, US, vol. 115, no. 11, 1 November 2005 (2005-11-01), pages 3117-3127, XP002448165 ISSN: 0021-9738
- MENGESHA ASFERD ET AL: "Potential and limitations of bacterial-mediated cancer therapy" FRONTIERS IN BIOSCIENCE, FRONTIERS IN BIOSCIENCE, ALBERTSON, NY, US, vol. 12, 1 January 2007 (2007-01-01), pages 3880-3891, XP002448166 ISSN: 1093-9946

## Description

The present invention relates to pharmaceutical compositions useful for the treatment of tumors, especially for the treatment of solid tumors. The present invention provides an improvement for known therapies using non-pathogenic bacteria for the treatment of solid tumors by providing the use of agents for the manufacture of pharmaceutical compositions that cause an increase of the penetration of the bacteria into tumor tissue.

### State of the art

Pawelek et al. ("Bacteria as tumor - targeting vectors", Lancet Oncology 4, 548-556 (2003)) describe that bacteria can be administered to organisms, e.g. human patients, suffering from a tumor, the administration of bacteria resulting in shrinkage of the tumor. Pawelek et al. found that tumor cells were destructed by the immunoreactions originally directed against the bacterial infection.

For tumor therapy using the administration of bacteria to the affected organism, it is known from Pawelek et al. that the natural preference of bacterial strains to target tumor tissue can be exploited for an improved therapeutic effect by manipulating the bacteria for the delivery of biologically active factors into the tumor tissue.

Dang et al. ("Combination bacteriolytic therapy for the treatment of experimental tumors", PNAS USA 98, 15155-15160 (2001)) demonstrated that bacteria accumulate and grow in necrotic regions of solid tumors, leaving a rim of viable tumor tissue.

The preference of obligate anaerobic bacteria, e.g. Clostridium and Bifidobacterium, for the hypoxic and anoxic regions of a solid tumor is explained by the preference of the bacteria for certain growth conditions (Bettegowda et aL, "Overcoming the hypoxic barrier to radiation therapy with anaerobic bacteria", PNAS USA 100, 15083-15088 (2003)). However, also facultative anaerobic bacteria used for tumor targeting, e.g. *Salmonella typhimurium* or *E. coli* were found not to migrate into viable tumor tissue, but remain in necrotic tumor regions (Forbes et al., "Sparse initial entrapment of systemically injected Salmonella typhimurium leads to heterogeneous accumulation within tumors", Cancer Research 63, 5188-5193 (2003)).

WO01/24637 describes a tumor treatment therapy by combined administration of bacteria and irradiation to the tumor. The bacteria can be genetically manipulated to produce a cellular toxin, e.g. colicin, or a cytokine or an anti-angiogenic protein.

WO 01/24637 A1 relates to a combination therapy of tumors by irradiation in combination with the administration of tumor targeted bacteria. The bacteria may include a cellular toxin.

### Objects of the invention

In view of the shortcomings of tumor therapies using bacteria for administration to a patient to achieve the reduction of viable tumor tissue, it is an object of the present invention to provide the use of compounds for the production of pharmaceutical compositions suitable for use in tumor therapy, which compositions improve the efficacy of the use of bacteria in tumor treatment, e.g. the use of compounds for pharmaceutical purposes in addition to or in combination with pharmaceutical compositions comprising bacteria, for use in tumor therapy.

### General description of the invention

The present invention achieves the above-mentioned objcets by the features of the claims, especially by providing the use of compounds for the production of pharmaceutical compositions for use in tumor therapy as well as a medical treatment in tumor therapy. Specifically, the present invention provides the use of a neutrophilic granulocyte diminishing and/or inactivating agent for the production of a pharmaceutical composition for use in bacterial tumor therapy, e.g. in combination with the use of bacteria for the production of a pharmaceutical composition for use in tumor therapy. Preferably, the neutrophilic granulocyte inactivating or diminishing agents are expressed by the bacteria used for the production of a pharmaceutical composition for bacterial tumor therapy.

Compounds and treatment according to the invention comprise the administration of non-pathogenic bacteria to an organism or patient bearing a solid tumor, the active compounds being specifically directed against the activity, preferably against the presence of neutrophilic granulocytes e.g. leading to the inactivation, reduction or depletion of neutrophilic granulocytes from the organism affected by the presence of a tumor, at least leading to the depletion of leukocytes within and/or around tumor tissue. For therapeutic purposes, non-pathogenic bacteria are used as a pharmaceutically active component, e.g. selected from attenuated bacteria, non-pathogenic bacteria and commensal bacteria. Exemplary bacteria are comprised in the group of gram-negative bacteria including *E. coli, Salmonella* spp., e.g. *Salmonella enterica* serovar Typhimurium, like strain SL7207, e.g. *Salmonella enterica* serovar Typhi, like strain Ty21a, *Shigella* spp., *Yersinia* spp., and *Vibrio cholerae* and gram-positive bacteria including *Bacillus* spp., e.g. *Bacillus subtilis, Clostridium spp., Listerium monocytogenes,* and *Mycobacterium* spp., e.g. strain BCG. Commensal bacteria are for example *E. coli, Lactobacillus* spp., *Lactococcus* spp., and *Streptococcus gordonii.* With reference to the affinity of *Vibrio cholerae* to tumors this is a property shared with at least some invasive bacteria, making them useful within the present invention. Non pathogenic bacteria for use in the present invention are comprised in the group including the following bacteria and, in the case of pathogenic bacteria, from respective attenuated strains thereof: *Agrobacterium* e.g. *Agrobacterium tumefaciens; Bacillus* e.g. *Bacillus cereus, Bacillus subtilis, Bacillus thuringiensis, Bacillus weihenstephanensis; Bartonella* e.g. *Bartonella henselae, Bartonella schoenbuchensis; Bdellovibrio* e.g. *Bdellovibrio bacteriovorus, Bdellovibrio starrii, Bdellovibrio stolpii; Bifidobacterium* e.g. *Bifidobacterium adolescentis, Bifidobacterium bifidum, Bifidobacterium lactis, Bifidobacterium longum; Bordetella* e.g. *Bordetella pertussis; Borrelia* e.g. *Borrelia burgdorferi; Brucella* e.g. *Brucella abortus, Brucella bronchiseptica; Burkholderia* e.g. *Burkholderia cenocepacia, Burkholderia fungorum, Burkholderia mallei, Burkholderia pseudomallei; Campylobacter* e.g. *Campylobacter fecalis, Campylobacter pylori, Campylobacter sputorum; Chlamydia* e.g. *Chlamydia pneumoniae, Chlamydia psittaci, Chlamydia trachomatis; Clostridium* e.g. *Clostridium difficile, Clostridium novyi, Clostridium oncolyticum, Clostridium perfringens, Clostridium sporogenes, Clostridium tetani; Corynebacterium* e.g. *Corynebacterium diphtheriae, Corynebacterium gluramicum, Corynebacterium jeikeium; Edwardsiella* e.g. *Edwardsiella hoshinae, Edwardsiella ictaluri, Edwardsiella tarda; Enterobacter* e.g. *Enterobacter aerogenes, Enterobacter cloacae, Enterobacter sakazakii; Enterococcus* e.g. *Enterococcus avium, Enterococcus faecalis, Enterococcus faecium, Enterococcus gallinarum; Escherichia* e.g. *Escherichia coli; Eubacterium* e.g. *Eubacterium lentum, Eubacterium nodatum, Eubacterium timidum; Helicobacter* e.g. *Helicobacter pylori; Klebsiella* e.g. *Klebsiella oxytoca, Klebsiella pneumoniae; Lactobacillus* e.g. *Lactobacillus bulgaricus, Lactobacillus casei, Lactobacillus delbrueckii, Lactobacillus plantarum; Lactobacterium* e.g. *Lactobacterium fermentum; Lactococcus* e.g. *Lactococcus lactis, Lactococcus plantarum; Legionella* e.g. *Legionella pneumophila; Listeria* e.g. *Listeria innocua, Listeria ivanovii, Listeria monocytogenes; Microbacterium* e.g. *Microbacterium arborescens, Microbacterium lacticum; Mycobacterium* e.g. *Bacille Calmette-Guérin (BCG), Mycobacterium avium, Mycobacterium bovis, Mycobacterium paratuberculosis, Mycobacterium tuberculosis; Neisseria* e.g. *Neisseria gonorrhoeae, Neisseria lactamica, Neisseria meningitidis; Pasteurella* e.g. *Pasteurella haemolytica, Pasteurella multocida; Salmonella* e.g. *Salmonella bongori, Salmonella enterica* ssp.; *Shigella* e.g. *Shigella dysenteriae, Shigella flexneri, Shigella sonnei; Staphylococcus e.g. Staphylococcus aureus, Staphylococcus lactis, Staphylococcus saprophyticus; Streptococcus* e.g. *Streptococcus gordonii, Streptococcus lactis, Streptococcus pneumoniae, Streptococcus pyogenes, Streptococcus salivarius; Treponema* e.g. *Treponema denticola, Treponema pallidum; Vibrio e.g.Vibrio cholerae; Yersinia* e.g. *Yersinia enterocolitica, Yersinia pseudotuberculosis,* including S1-strains devoid of Hfr factors and of pili of these bacteria, especially S1-strains ofE. *coli.*

For the present specification, the term leukocyte generally comprises the group of granulocytes like neutrophilic, basophilic and eosinophilic granulocytes and mast cells. In addition to granulocytes, leukocytes can also include macrophages and dendritic cells.

The present disclosure is based on the observation that the depletion or inactivation of leukocytes in and/or around tumor tissue results in an infiltration of the entire tumor tissue, including the viable tumor tissue, by bacteria administered for therapeutic purposes, resulting in a significant improvement of tumor size reduction.

Further, it has been shown that the depletion of leukocytes, more preferred of granulocytes, and most preferred of neutrophilic granulocytes, essentially from the entire organism affected by the presence of a tumor does not significantly impair the health status of the organism or patient, and leads to a significant improvement of bacterial tumor therapy, namely to a significant reduction of tumor tissue, especially of viable tumor tissue, by the presence of bacteria used for therapy essentially throughout the entire tumor.

In the alternative to a systemic inactivation or depletion of leukocytes, the depletion or inactivation of leukocytes, more preferred of granulocytes, and most preferred of neutrophilic granulocytes can be effected essentially for the tumor tissue only, e.g. as a local depletion or inactivation of these immune cells in the vicinity of and/or within a solid tumor.

Initially, it was observed that the administration of bacteria for therapeutical purposes in tumor therapy essentially leads to colonization of the necrotic regions of tumor tissue, while leaving the viable regions of tumor tissue essentially unaffected. As a result, tumor therapy using bacteria to-date leaves at least a fraction of the viable tumor tissue intact.

A cause for the limitation of the spread of bacteria used in tumor therapy to necrotic regions of a solid tumor is believed by the present inventors to be caused by a barrier generated by leukocytes, especially of neutrophilic granulocytes and macrophages, gathering at a boundary or interfacial area of viable tumor tissue towards necrotic regions and towards healthy tissue.

The presence of an agent for the inactivation, reduction in number, i.e. diminishing leukocytes, or depletion of leukocytes by at least 50%, preferably by at least 80%, more preferably by at least 95%, most preferably by about 99%, especially of neutrophilic granulocytes and macrophages, results in the colonization of viable tumor tissue by bacteria administered for therapeutic purposes in tumor therapy. As a consequence of the inactivation, reduction in number and/or depletion of leukocytes, therapeutically administered bacteria can locate into the viable tumor tissue, which leads to a significant reduction, preferably to the elimination of viable tumor tissue.

Surprisingly, it could be shown that the depletion of a fraction of the natural immune system, namely of leukocytes, especially of granulocytes, more especially of neutrophilic granulocytes by at least 50% and up to more than 90% from the blood of an organism affected by the presence of a tumor leads to a significantly higher efficacy of tumor reduction by bacterial treatment but does not result in significant impairments of the organism. Accordingly, it is demonstrated by the present invention that the virtual elimination of leukocytes, more especially of neutrophilic granulocytes, from the blood of an organism results in an increased efficacy of tumor therapy using the administration of bacteria. Preferably, the number of leukocytes, more especially of neutrophilic granulocytes or macrophages is reduced, more preferably depleted from the tumor bearing organism, preferably from the tumor tissue only and, more preferably, for a limited period of time, allowing the restoration of the presence of leukocytes, more especially of neutrophilic granulocytes or macrophages after a significant decrease of viable tumor tissue, preferably after complete destruction of the tumor tissue. Accordingly, it is preferred that the compounds for inactivation or depletion of leukocytes, more especially of neutrophilic granulocytes or macrophages are used for the production of a pharmaceutical composition for use in the treatment of solid tumors for transient inactivation or depletion.

For temporary depletion of leukocytes, the present specification in a first aspect provides the use of antibodies, specifically directed against leukocytes, especially antibody specific for granulocytes or macrophages, for the production of pharmaceutical compositions for use in tumor therapy, for local or systemic administration. Administration of antibodies specific against leukocytes leads to their depletion from the blood of the organism affected by presence of a tumor. In the alternative or in addition to the administration of an antibody preparation, antibody can be provided by expression by the bacteria used in tumor therapy, which bacteria are genetically manipulated to express an antibody directed specifically against leukocytes, preferably including the secretion of the antibody in soluble form.

In addition to or in the alternative to the use of antibodies directed against leukocytes, the presence of leukocytes in and around tumor tissue can significantly be reduced by eliminating for instance the activity of cytokines and/or chemokines, which attract leukocytes to the tumor tissue. Accordingly, the present specification provides the use of antibody specifically directed against a cytokine or a chemokine attracting leukocytes for the production of a pharmaceutical composition, in combination with the use of bacteria in a pharmaceutical composition against tumor, because the compounds masking or otherwise eliminating the activity of cytokines and/or chemokines attracting leukocytes to the surrounding of or into tumor tissue also result in an increased presence of the bacteria used in tumor therapy within the viable regions of tumor tissue and, as a consequence, in an increased reduction of viable tumor tissue.

In addition to or in the alternative to the administration of compounds, e.g. antibody, eliminating the activity of cytokines and/or chemokines attracting leukocytes to the site of tumor tissue, by administration of antibody directed against the specific leukocytes attracting cytokines and/or chemokines, to the organism affected by a tumor, these antibodies specifically directed against chemokines and/or cytokines can be provided by expression by the bacteria used in tumor therapy, which bacteria are genetically manipulated to express the specific antibodies, preferably in soluble form and including secretion.

Accordingly, the present specification in addition to providing the use of antibody specifically directed against leukocytes and/or the use of antibody specifically directed against the activity of cytokines and/or the use of antibody specifically directed against chemokines attracting leukocytes within tumor tissue, for use in the preparation of a pharmaceutical composition for tumor therapy, and the respective tumor therapy, the present invention provides bacteria for use in the production of pharmaceutical compositions for tumor therapy, which bacteria are genetically manipulated to express antibody directed against leukocytes and/or antibody directed against cytokines and/or antibody specifically directed against chemokines attracting leukocytes. For the purposes of this invention, the expression of anti-leukocyte specificity can be used to include both toxins specifically directed against leukocytes, antibody specifically directed against leukocytes, and antibody directed against a cytokine and/or against a chemokine having leukocyte attracting properties. Expression of at least one of these antibodies by the bacteria used for tumor therapy predominantly leads to presence of these antibodies within the tumor tissue and its surroundings because the bacteria used in tumor therapy have a natural preference for the tumor tissue, e.g. based on the facultative anaerobic or obligate anaerobic habitat requirements.

For suppressing the activity of cytokines and/or chemokines, antibodies can be used for the production of a pharmaceutical composition according to the invention, which antibodies are comprised in the group of antibody neutralizing tumor necrosis factor alpha (TNFα), antibody neutralizing interleukin 8 (IL-8), antibody neutralizing epithelial-derived neutrophil attractant (ENA-78, corresponding to CXCL5), antibody neutralizing growth - related oncogene alpha (gro-α), antibody neutralizing interferon-γ-inducible-lymphocyte-attractant chemokine (monocyte chemoattractant protein 1, MCP 1), antibody neutralizing interferon gamma inducible protein (IP - 10), antibody neutralizing monokine induced by interferon gamma (MIG) and/or antibody neutralizing formyl-MLP, antibody neutralizing anaphylatoxin C5a, antibody neutralizing anaphylatoxin C3a, antibody neutralizing prostaglandines, e.g. antibody neutralizing prostaglandine E1, antibody neutralizing prostaglandine MIP-1α (macrophage inflammatory protein 1β), antibody neutralizing prostaglandine MIP-1β (macrophage inflammatory protein 1α), antibody neutralizing RANTES/CCL5 (RANTES = regulated upon activation , normal T-cell expressed and secreted), and/or antibody neutralizing leukocyte adhesion factor LFA-1. These antibodies can be generated according to standard procedures as a polyclonal serum fraction, or as a monoclonal preparation or, preferably, by expression from the nucleic acid sequence encoding the antibody in a manipulated bacterium used in the pharmaceutical composition for tumor therapy.

Antibodies suitable for depleting leukocytes can be selected from the group comprising anti-CD11b, anti-CD11c, anti-Gr1 and anti-F4/80.

For systemic depletion of monocytes, which are precursor cells of macrophage and granulocytes, chlodronate, e.g. formulated into a liposome preparation, can be used.

Further, the depletion or inactivation of leukocytes can be effected by genetically manipulating bacteria used in tumor therapy to express a cytotoxin, e.g. colicin or *Pseudomonas* exotoxin. Alternatively, the depletion or inactivation of leukocytes can be caused using natural bacteria having cytotoxic activity, e.g. *E. coli* of the phylogenetic group B2, which express a cytotoxin. Accordingly, the present specification provides the use of cytotoxin expressing bacteria for the production of a pharmaceutical composition for tumor therapy.

Preferably, the cytotoxin has a preference for inhibiting or depleting leukocytes and a less pronounced effect on other cells like e.g. erythrocytes. More preferably, the cytotoxin is specific for leukocytes. In accordance with the preference for an anti-leukocyte specificity of the cytotoxin for use in the preparation of a pharmaceutical composition according to the present invention, the cytotoxin is provided for administration or activity at the site of leukocyte accumulation in the vicinity of the tumor or, more preferably, the cytotoxin does not or only to a limited extent have general cytotoxic activity directed against all cells, but has anti-leukocyte specificity. Exemplary preferred leukocyte specific cytotoxins are comprised in the group of a soluble cytotoxin obtainable from *Pasteurella haemolytica* culture supernatant as described by Shewen et al. (Infection and Immunity, 91-94 (1982)), staphylococcal leukocidin, soluble leukocyte toxin obtainable from *Actinobacillus actinomycetemcomitans* as described by Tsai et al. (Infection and Immunity 427-439 (1979)), and leukocidins, e.g. Panton-Valentine leukocidin *of Staphylococcus aureus*, described by Genestier et al. (The Journal of Cinical Investigation 3117-3127 (2005)).

Bacteria for use as a pharmaceutical agent in tumor therapy comprising an expression cassette for a synthesis product essentially depleting or at least inactivating leukocytes, e.g. selected from the aforementioned antibodies or toxins, especially those having specificity against neutrophilic granulocytes or macrophages, can comprise a constitutive promoter for expression control, preferably an inducible promoter being inducible by an agent that can be administered separate from the bacteria to the organism affected by presence of the tumor. A preferred inducible promoter for the expression cassette for the anti-leukocyte synthesis product is a saccharide inducible promoter, especially the arabinose inducible promoter. For release of the toxin and/or antibody directed against leukocytes, the genetically manipulated bacteria comprising the coding sequences for the anti-leukocyte toxin and/or anti-leukocyte antibody can be provided with coding sequences and/or regulatory sequences providing for the secretion of the anti-leukocyte toxin and/or anti-leukocyte antibody from the bacteria, or for release of the anti-leukocyte toxin and/or anti-leukocyte antibody by inducible lysis of the bacteria.

Preferably, the anti-leukocyte antibody is a single-chain antibody designed for synthesis in its active form, e.g. in soluble form, in the bacterium. As an example, the anti-leukocyte antibody is devoid of a constant chain domain and/or devoid of a light chain domain, e.g. the antibody is a minibody or nanobody, e.g. corresponding to the structure of camelids, which are antibodies obtainable from camel and alpaca.

As a further aspect, bacteria for use in tumor therapy comprising an expression cassette, the synthesis product of which has anti-leukocyte activity, e.g. leads to the reduction of leukocytes, especially preferred to the depletion of leukocytes, also contain an expression cassette for a translation product having anti-tumor activity, the expression cassette preferably under the control of an inducible promoter, which preferably is a tumor-specific promoter, which is more preferably inducible separately from the promoter controlling the expression cassette encoding the anti-leukocyte agent.

### Detailed description of the invention

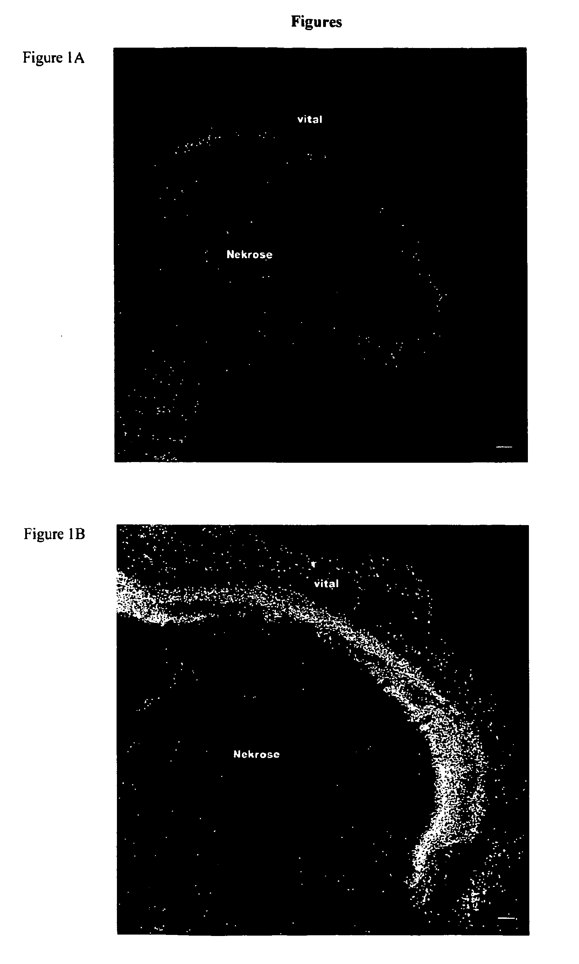

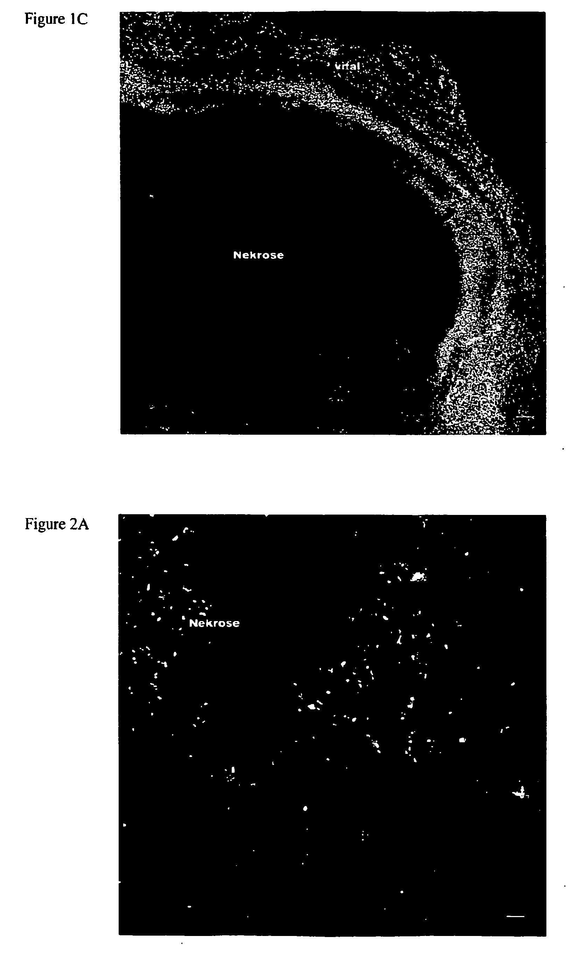

The present invention is now described in greater detail by way of examples with reference to the figures, wherein
- Figures 1 A, B and C for comparison show microscopic pictures of one sample section of a mouse tumor after systemic infection with *Salmonella typhimurium* in the identical sample section, namely at A) the localization of *Salmonella typhimurium,* at B) the localization of neutrophilic granulocytes, and at C) the localization of macrophages, with the white bar on the bottom right corner representing 100 µm,

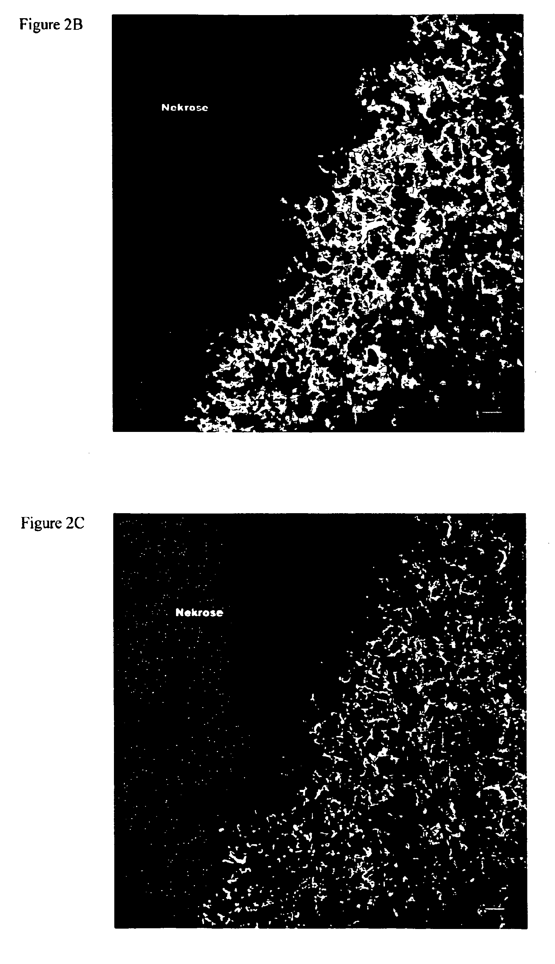

- Figures 2 A, B and C show enlargements of the microscopic pictures of Figure 1 in one sample section, namely at A) the localization of *Salmonella typhimurium,* at B) the localization of neutrophilic granulocytes, and at C) the localization of macrophages, with the white bar on the bottom right corner representing 10 µm,

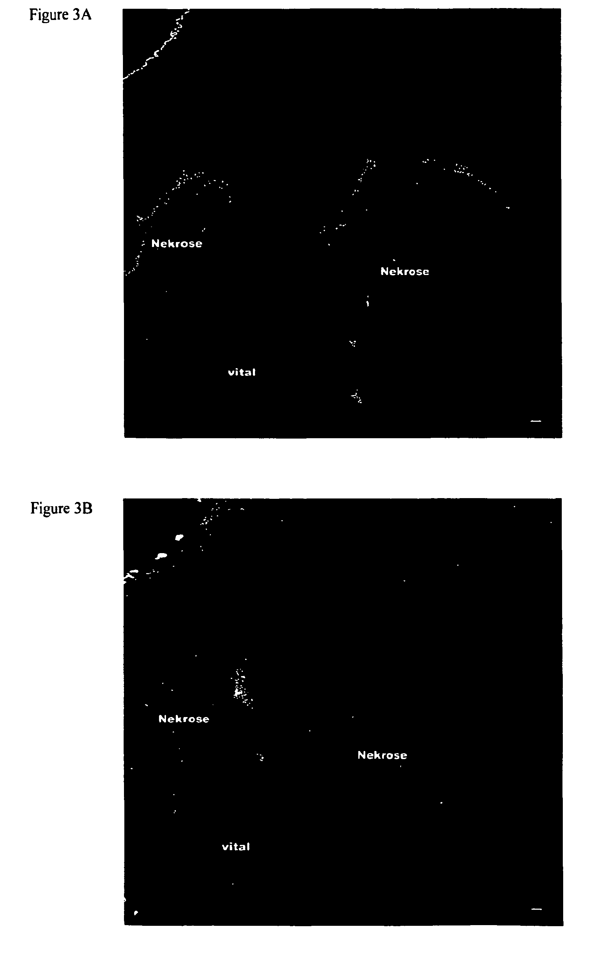

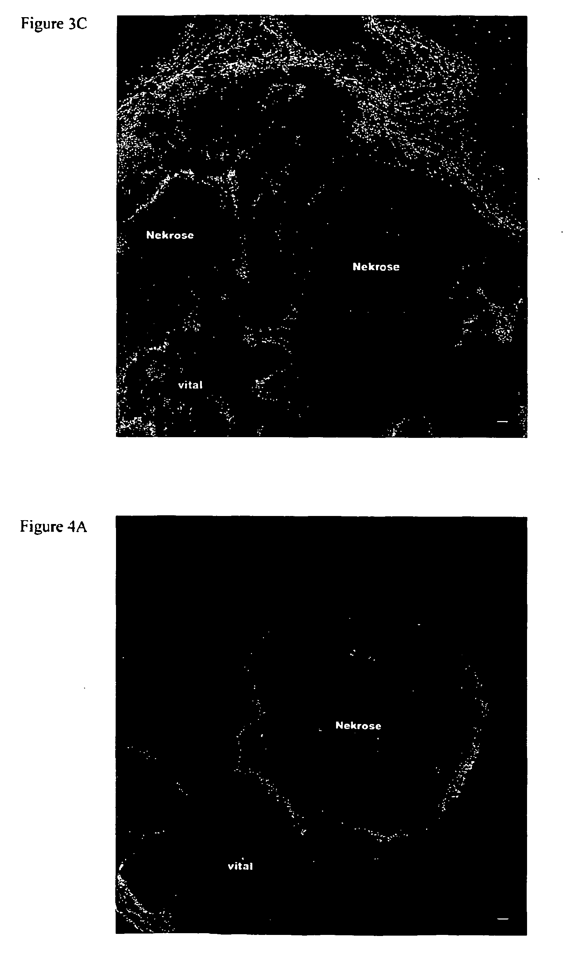

- Figures 3 A, B and C show microscopic pictures of one sample section of a mouse tumor after systemic depletion of neutrophilic granulocytes, followed by systemic infection with *Salmonella typhimurium* shortly after bacterial infection in one sample section, namely at A) the localization of *Salmonella typhimurium,* at B) the localization of neutrophilic granulocytes, and at C) the localization of macrophages, with the white bar on the bottom right corner representing 100 µm,

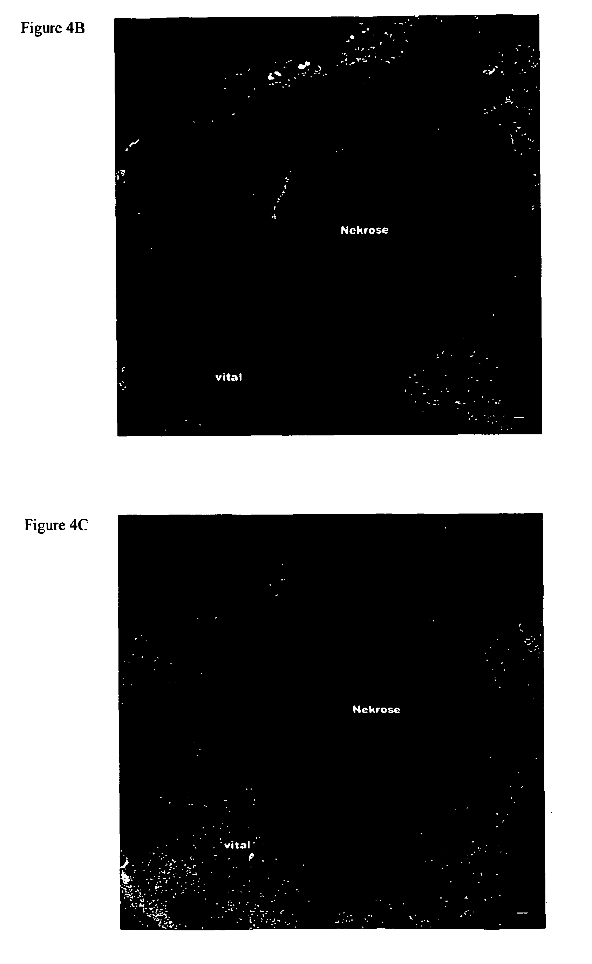

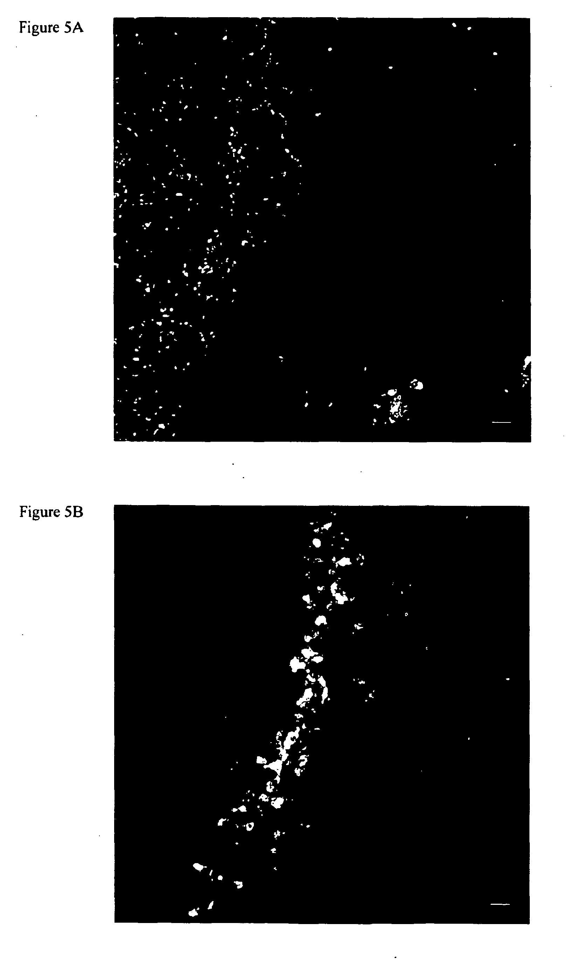

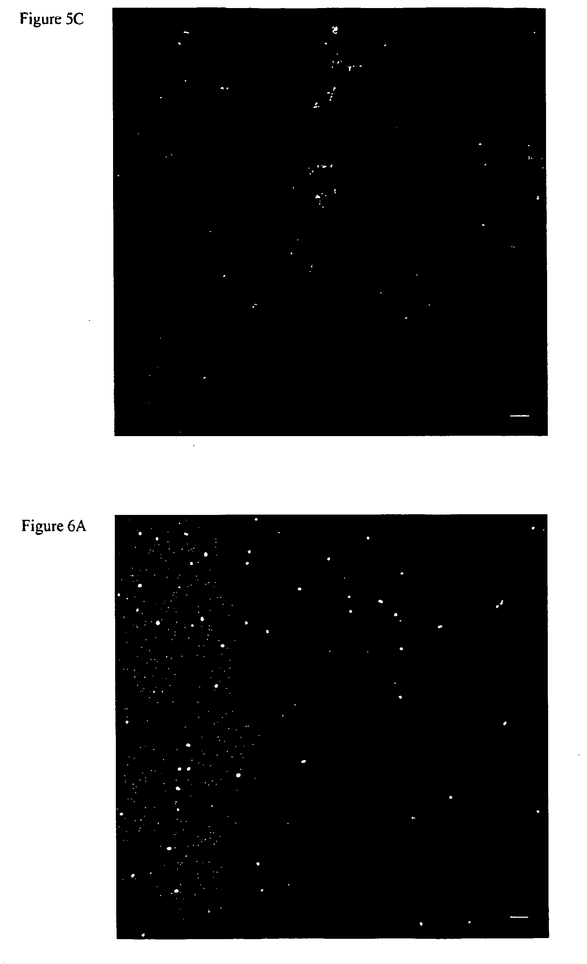

- Figure 4 A, B and C show microscopic pictures of one sample section of a mouse tumor after systemic depletion of neutrophilic granulocytes, in which depletion of neutrophilic granulocytes was achieved only to 95%, followed by systemic infection with *Salmonella typhimurium* shortly after bacterial infection in a similar sample section, namely at A) the localization of *Salmonella typhimurium,* at B) the localization of neutrophilic granulocytes, and at C) the localization of macrophages, with the white bar on the bottom right corner representing 100 µm, Figures 5 A, B and C show microscopic pictures of one sample section of a mouse tumor after systemic depletion of neutrophilic granulocytes, followed by systemic infection with *Salmonella typhimurium* 2 days post bacterial infection in enlargement from Figure 4 in an identical sample section, namely at A) the localization of bacteria, at B) the localization of neutrophilic granulocytes, and at C) the localization of macrophages, with the white bar on the bottom right corner representing 10 µm,

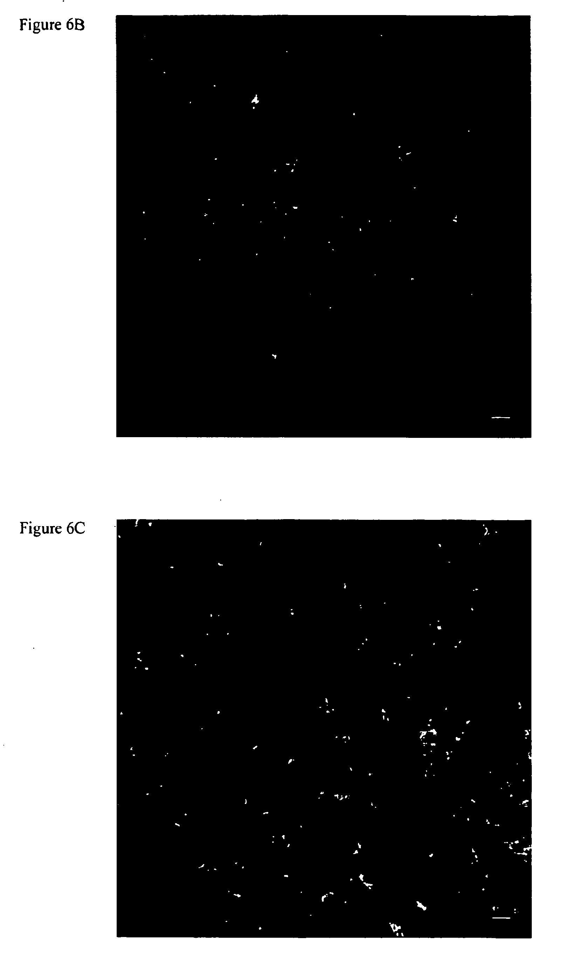

- Figures 6 A, B and C show microscopic pictures of vital tumor tissue of a mouse tumor after systemic depletion of neutrophilic granulocytes, followed by systemic infection with *Salmonella typhimurium* 2 days post bacterial infection in an identical sample section, namely at A) the localization of *Salmonella typhimurium,* at B) the localization of neutrophilic granulocytes, and at C) the localization of macrophages, with the white bar on the bottom right corner representing 10 µm,

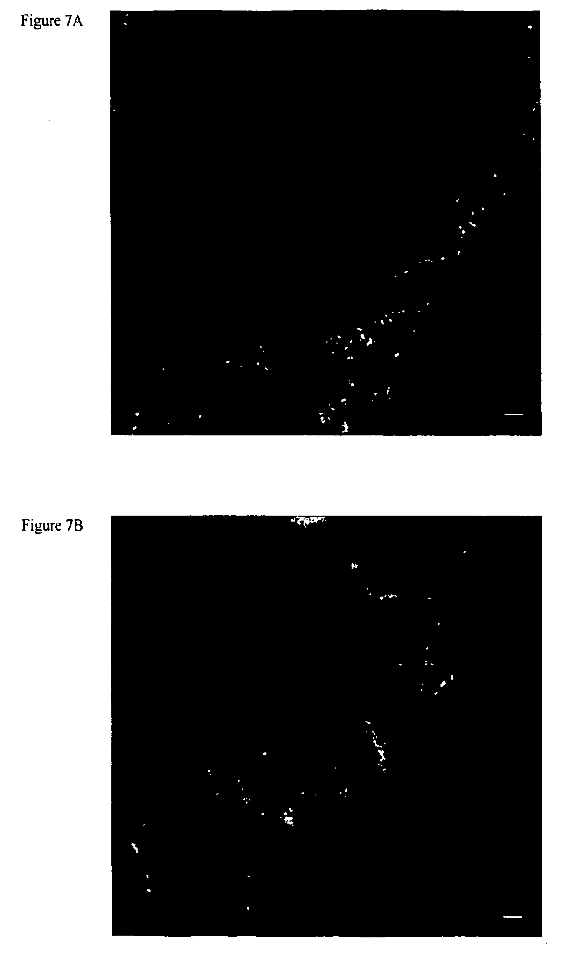

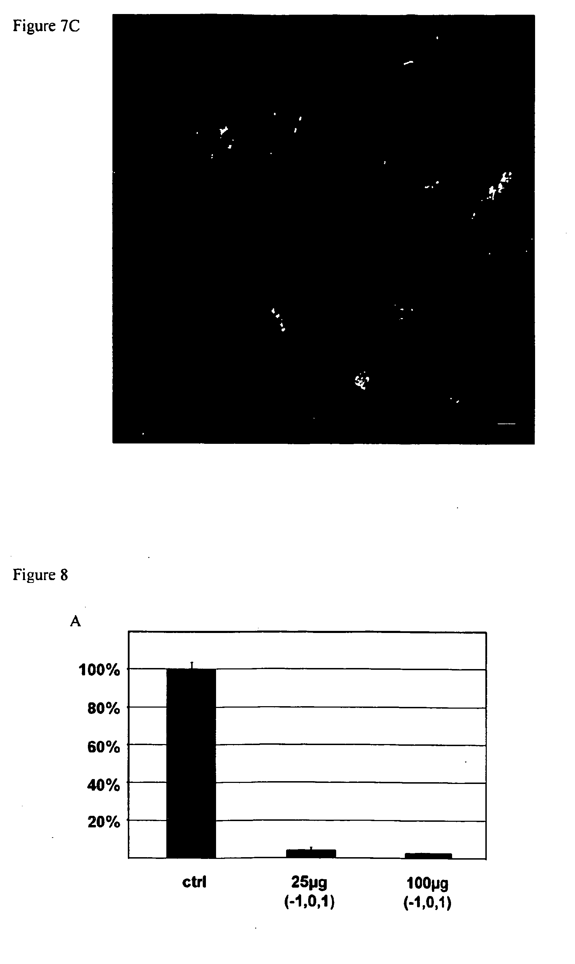

- Figures 7 A, B and C show microscopic pictures of a vital region of a mouse tumor directly at the border of the tumor next to the skin after systemic depletion of neutrophilic granulocytes, followed by systemic infection with *Salmonella typhimurium* 2 days post bacterial infection, namely at A) the localization of *Salmonella typhimurium,* at B) the localization of neutrophilic granulocytes, and at C) the localization of macrophages, with the white bar on the bottom right corner representing 10 µm, and

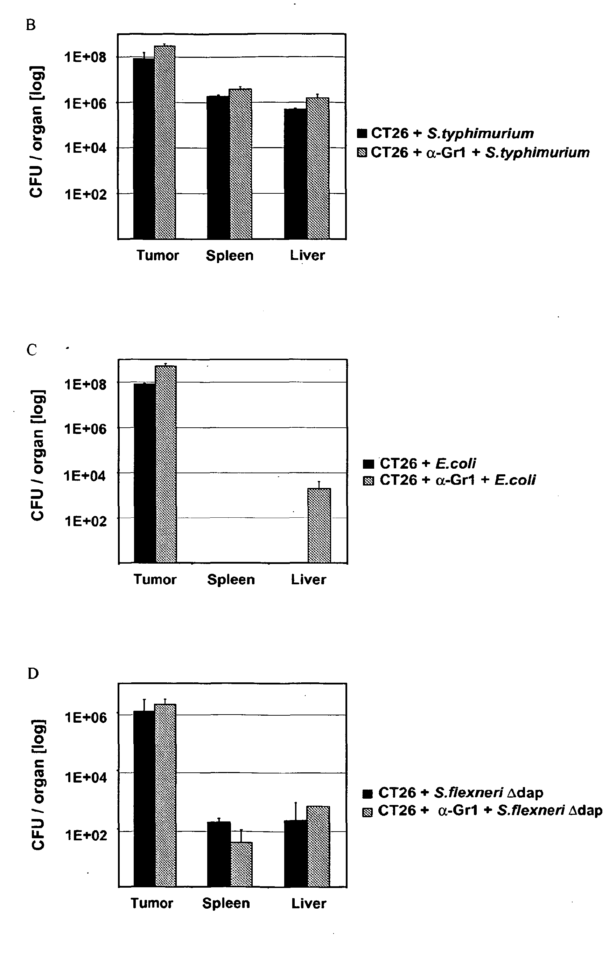

- Figure 8 shows results of A) neutrophil counts after administration of antibody depleting neutrophilic granulocytes, B) bacterial counts of *S. typhimurium,* C) bacterial counts of *E. coli,* and D) bacterial counts of *Shigella flexneri,* after administration of these bacteria to separate experimental animals, with black columns referring to bacterial counts in experiments according to the invention after administration of a granulocyte specific antibody for depletion of neutrophils, and comparative grey columns referring to bacterial counts without the depletion of neutrophilic granulocytes.

Micrographs of Figures 1 to 7 were taken from cryosections of 10 µm thickness prepared from snap frozen tumor tissue (Tissue-Tek OCT compound, obtained from Sakura Finetek) from sacrificed mice using a microtome cryostat (Cryo-Star HM 560V, Microm), followed by air-drying at room temperature overnight and fixing in acetone at -20 °C for three min, rehydrating in PBS, blocking with 50 µg/mL BSA and 1 µg/mL FcR blocker (rat anti-mouse CD 16/CD 32). For specific staning of *Salmonella typhimurium,* polyclonal rabbit anti-*S. typhimurium* (Sifin) and polyclonal goat anti-rabbit Alexa 488 (Sigma) were used, for staining of *Shigella flexneri*, polyclonal got anti-rabbit with Alexa 488 (Sigma) and polyclonal rabbit anti-*Shigella flexneri* (Biomol) were used, for staining of *E. coli,* polyclonal goat-anti-*E. coli* (Biomol) and polyclonal rabbit anti-goat Alexa 488 (Invitrogen) were used.

For staining of neutrophilic granulocytes, rat-anti-Gr1 biotin (RB6-8C5) and streptavidin-cy5 (Molecular Probes) for staining of macrophages rat anti-CD 11b PE (eBioscience) were used, and for staining of eucaryotic cells, Phalloidin Alexa fluor 594 (Molecular Probes) and DRAQ5 (Biostatus) were used. After staining, the slides were washed and dried, mounted with mounting medium (Neomount, Merck) and analysed using a laser scanning confocal microscope (LSM 510 Meta, Zeiss) followed by image processing using an LSM5 image browser (Zeiss) and Adobe photoshop 7.0.

For paraffin sections, tumors were fixed with 10% (v/v) paraformaldehyde and imbedded in paraffin wax. Sections of 5 µm were mounted starfrost slides and stained with hematoxilin and eosin. Stained paraffin sections were analysed with an Olympus BX51 microscope.

In Figures 1 to 7, similar sample sections are shown, respectively. Accordingly, superimposition of Figures A - C allows to determine the relative localization of leukocytes and bacteria after their specific detection. Necrotic tissue (indicated as "Nekrose") and vital tumor tissue (indicated as "vital") were detected by specific staining and/or light microscopy.

### Comparative example 1: Use of bacteria for the treatment of tumors in mice

For comparative purposes, BALB/c mice (6 weeks old, female, purchased from Harlan, Borchen, Germany) were subcutaneously inoculated at the abdomen with 5 x 10⁵ cells of the colon adenocarcinoma cell line CT26 (available as ATCC CRL-2638), which were grown as monolayers in IMDM medium (Gibco BRL), supplemented with 10 % (v/v) heat inactivated fetal calf serum, 250 µM β-mercapto ethanol and 1% (v/v) penicillin - streptomycin.

After 10 days following injection, mice bearing tumors of diameters from 5 to 7 mm were infected with bacteria suspended in phosphate buffered saline (PBS) using 5 x 10⁶ cfu of *Salmonella typhimurium* (strain SL2707, hisG, ΔaroA (Hoiseth and Stocker, 1981)) or *E. coli* TOP10 (Invitrogen, Karlsruhe, Germany) from overnight cultures grown at 37 °C in shake flasks intravenously, or intratumorally with 1x 10⁷ *Shigella flexneri* (sero type 5, Δdap, according to Sansonetti et al., 1982), grown in tryptic soy broth, supplemented with 200 µM Congo red, 30 µg/mL kanamycin, 100 µg/mL DAP at 37 °C in shake flasks.

The intratumoral administration of *Shigella flexneri* was used because initial experiments showed that systemic administration of *Shigella flexneri* did not result in a preferential accumulation of the bacteria in tumor tissue, as was observed for salmonella and *E. coli.*

In Figure 1 A - C and its enlargement in Figure 2 A - C, sections of mouse tumor two days post systemic bacterial infection by *Salmonella typhimurium* and without depletion of leukocytes are shown. The accumulation of macrophages (Figures 1C and 2C) in one line can be seen, corresponding to the localization of neutrophilic granulocytes (Figures 1B and 2B), and which can be interpreted as the accumulation of leukocytes in one plane. In vital tumor tissue (indicated by "vital") identified in light microscopy, no significant presence ofbacteria (Figures 1A and 2A) was detected whereas bacteria could be localized in necrotic regions (indicated as "Nekrose").

Analyses of tumor tissue from mice after infection with *E. coli* or after infection with *Shigella flexneri*, show that bacteria are essentially limited to necrotic regions of the tumor with a layer of leukocytes being arranged between necrotic and viable tumor tissue.

### Example 1: Use of a granulocyte - specific antibody for the production of a pharmaceutical composition for the depletion of neutrophilic granulocytes in combination with artificial bacterial infection

Using the experimental procedure of comparative example 1, a significant depletion of neutrophils according to the invention was initiated by administering three doses of 25 µg each of monoclonal rat-anti-Gr1 (RB6-8C5) antibody, diluted in 100 µL PBS intraperitoneally, one day before, simultaneously, and 1 day following bacterial infection.

In Figures 3 to 7, cryosections are depicted, now showing detection of the bacteria in trial dispersed throughout the tumor tissue, including the viable regions of the tumor. In detail, Figures 3-7 show the effect of the depletion of neutrophilic granulocytes as a significantly reduced number of neutrophilic granulocytes (Figures 3 and 4 B) from tumor tissue and the destruction of the layer formed by neutrophilic granulocytes at the interfacial area between necrotic and viable tumor tissue. Correspondingly, the number of leukocytes detected in tumor tissue is reduced.

In detail, the enlargement of a fraction of Figure 4, shown as Figure 5, demonstrates that following the depletion of leukocytes, especially of neutrophilic granulocytes from tumor tissue results in the spread of bacteria that were administered systemically or locally to tumor tissue also into viable tumor tissue because bacteria can be detected on both sides of the interface between necrotic and viable tumor tissue, even if a fraction of leukocytes can be detected to remain at this interface.

In Figure 6 bacteria are now be detected also to reside in vital tumor tissue. This ie corroborated by Figure 7 showing vital tumor tissue at the border of the tumor and skin, in which vital tumor tissue now bacteria are found.

When comparing the extension of the necrosis observed by microscopy, a significant increase of the necrotic area for the artificially induced tumor could be detected caused by the depletion of neutrophils, exemplified here by the application of the granulocyte specific antibody α- Gr1. Results are summarized in table 1.

**Table 1: Percentage necrosis after administration of bacteria with and without the depletion of neutrophils**

| tumor | bacteria administered | depletion with α-Gr1 antibody | necrosis [%] |
|---|---|---|---|
| CT26 | No infection | - | 5-15 |
| | *S. typhimurium* SL7270 | - | 60-65 |
| | *S. typhimurium* SL7270 | + | 75-85 |
| | *E. coli* TOP 10 | - | 60-65 |
| | *E. coli* TOP 10 | + | 80-85 |
| | *S. flexneri* Δdap | - | 65-70 |
| | *S. flexneri* Δdap | + | 85-90 |

Table 1 shows that in addition to the increase of necrotic area within a solid tumor, an increase in the number of viable bacteria per volume of tumor tissue is observed, demonstrating the increased colonization of the tumor by bacteria effected by the reduction of the number of neutrophilic granulocytes, preferably by their depletion.

Figure 8 A shows the influence of the experimental systemic administration of the neutrophilic granulocyte-specific antibody rat-α- Gr1 RB6-8C5) to control animals without any treatment (ctrl), with administration of 25 µg antibody 1 day before administration of the bacteria, (-1), concurrent with the administration of bacteria (0) and 1 day following bacterial administration (1), and for doses of 100 µg antibody, respectively. It can be seen that neutrophils are drastically reduced already by doses of 25 µg antibody per mouse and even further by doses of 100 µg.

Neutrophilic granulocytes were counted by flow cytometry, using 50 µL blood, lysis in 1.5 mL erythrocyte lysis buffer, vortexing, incubating for 5 minutes at room temperature and centrifuging for 5 minutes. The lysis procedure was repeated once. Cell pellets were washed once with PBS and stained with rat-α-Gr1 FITC (RB6-8C5) and with rat-α-CD11b PE (eBioscience) for 20 minutes on ice. After staining, cells were washed with PBS and analyzed by cell sorting (FACS) (FACSCalibur, Becton Dickinson).

When analyzing the colonization of tumor, spleen and liver after the depletion of neutrophilic granulocytes by administration of rat-α-Gr1 antibody (black columns) in comparison to the bacterial treatment without depletion of neutrophilic granulocytes, (grey columns), a drastic increase of colony forming units (CFU) that could be counted after plating the respective tissue homogenates from artificially infected mice demonstrates the effective colonization of tumor tissue caused by the administration of compounds for reducing or depleting neutrophilic granulocytes from the tumor affected organism or patient.

In the alternative to the use of an anti-granulocyte antibody for producing a composition for tumor therapy, the spread of bacteria into viable tumor tissue was equally enhanced by depleting leukocytes by making use of chlodronat. For depletion of macrophages by chlodronat, chlodronat-containing liposomes were administered at doses of 2,5g chlondronat/kg body weight. Following the depletion of macrophages, the inoculation of the experimental animals was made as described above.

Results of bacteria spreading into viable tumor tissue and bacterial counts within tumor tissue and liver and spleen were comparable to results obtained with anti-granulocyte antibody. Further, reductions of tumor size were similar to those obtained with the anti-granulocyte antibody.

An observation of the health status of mice after depletion of macrophages did not show a significant impairment of the general health status, although mice were found to have reduced activity levels.

### Example 2: Use of bacteria constitutively expressing anti-neutrophilic granulocyte antibody for the production of a composition for tumor therapy

As an alternative to the separate administration of an agent directed to substantially decrease the number of neutrophilic granulocytes, bacteria suitable for tumor therapy were genetically manipulated to express and secrete an anti-granulocyte antibody, namely a soluble form of rat-α-Gr1 (RB6-8C5) antibody. For the expression of the anti-granulocyte antibody, bacterial cells were transformed using a pBR322-derived bacterial plasmid containing an expression cassette, constitutively expressing the anti-granulocyte antibody under the control of the promoter of the *E. coli* β-lactamase gene (P_{bla}).

The administration procedure of Example 1 of mice bearing an artificially induced CT26 tumor, with *Salmonella typhimurium* SL7207, *E. coli* TOP 10 or *Shigella flexneri* (Δdap), respectively, was repeated after transformation of the bacteria with the expression plasmid encoding the soluble rat-α-Gr1.

Analysis of the tumor tissue showed that bacteria had spread throughout the tumor tissue, including the viable regions of the tumor. The anti-tumor effect was increased in comparison to the same bacterial strains lacking the constitutive expression cassette for the anti-granulocyte antibody, reaching approximately the increase in necrotic area as obtained in Example 1 using separate administration of the anti-granulocyte antibody.

### Example 3: Use of bacteria inducibly expressing anti-neutrophilic granulocyte antibody for the production of a composition for tumor therapy

As a further the embodiment of the invention, the constitutive promoter of the expression cassette of Example 2 was exchanged for a saccharide inducible promoter, namely the *E. coli* arabinose- inducible promoter P_{BAD}. The P_{BAD} promoter has the advantage of being closely regulated in the absence of the inductor saccharide L-arabinose and allowing rapid induction of protein synthesis in the presence of the inductor saccharide, while the inductor saccharide can be administered to the tumor bearing organism or patient separate from the genetically manipulated bacteria.

In this example, bacteria harboring the expression cassette for encoding the anti-neutrophilic granulocyte antibody under the control of the P_{BAD} promoter were administered to tumor bearing mice. After 2 days following administration of the bacteria, arabinose as the suitable inductor saccharide was administered in an amount of 5 g/kg body weight. Following the administration of the inductor saccharide, analysis of the tumor tissue after 2 days showed an effective colonization of the entire tumor tissue, including its viable regions, with the bacteria.

The reduction in tumor size was approximately equally effective as in Examples 1 and 2.

### Example 4: Bacterial vector for use in tumor therapy comprising an expression cassette for an anti-neutrophilic granulocyte antibody and an inducible expression cassette for an anti-tumor toxin

For increasing the efficacy of bacterial presence in the viable portions of tumor tissue according to the invention, the bacteria used for tumor therapy in addition to an expression cassette encoding an anti-neutrophilic granulocyte antibody were transformed with an expression cassette derived from pACYC184 (GenBank/EMBL accession number X06403) encoding a cytotoxin under the control of an inducible promoter. The inducible promoter for cytotoxin synthesis according to a preferred embodiment is also a saccharide inducible promoter, e.g. the *E. coli* arabinose- inducible promoter P_{BAD} or the *E. coli* rhamnose-inducible promoter Pᵣₕₐ.

A bacterial pBR322-derived plasmid containing both an expression cassette for anti-leukocyte antibody, namely for a soluble variant of rat-α-CD11b, and an expression cassette for a cytotoxin, namely a colicin. When inducible promoters, preferably saccharide-inducible promoters responding to different inductors are arranged before the structural genes within the expression cassettes for the leukocyte diminishing or inactivating agent and the toxin, respectively, expression of both molecules can be induced separately. Separately inducible expression cassettes are advantageous because separate induction of the leukocyte diminishing or inactivating agent and the toxin, respectively allows to better control the anti-tumor effect of the bacteria. Plasmid construction was according to standard cloning procedures.

Further examples for cytotoxins that can be encoded in an expression cassette within bacteria for use in tumor treatment of the invention are comprised in the group of colicins, pseudomonas exotoxins.

### Example 5: Bacteria for use in tumor therapy encoding a cytotoxin against eukaryotic cells

Local depletion of leukocytes in tumor tissue and reduction of tumor size, including the reduction of viable tumor tissue, was obtained by systemic administration of bacteria naturally expressing a cytotoxic activity or genetically manipulated to express a cytotoxin active against eukaryotic cells. The bacteria were preferably *E. coli* selected from the phylogenetic group B2, which naturally express a cytotoxic activity. In the alternative, an expression cassette encoding the cytotoxic activity was synthesized and functionally linked to a bacterial promoter, then integrated into a non-toxin bearing bacterial strain as a plasmid or integrated into the bacterial genome.

Cytotoxic *E. coli* were administered at 10⁶ cfu systemically to CT26 tumor bearing BALB/c mice, generated according to Comparative Example 1. Histological analysis was done on day three and tumor growth was monitored for 10 days following bacterial infection. For comparison, toxin-free *E. coli* TOP10 was used in control animals.

Analyses showed that the administration of cytotoxic activity bearing bacteria in comparison to the control animals receiving toxin-free bacteria resulted in a significantly improved retardation of tumor growth, preferably in a significant reduction of tumor size, especially in a reduction of viable tumor tissue. Further, it was shown that leukocytes were reduced in number, preferably depleted from tumor tissue.

Presently, it is believed that the anti-tumor effect of the use of bacteria expressing a cytotoxic compound as an anti-tumor agent, exemplified here by the cytotoxic *E. coli* IHE 3034 (group B2) is based on the activity of the hybrid peptide-polyketide genotoxin, known to induce DNA double-strand breaks and an activation of the DNA damage checkpoint pathway (Nougayrede et al., Science 313, 848-851 (2006)), resulting in the desired reduction of tumor growth and tumor size.

## Claims

1. Pharmaceutical composition for use in the therapy of solid tumors, which therapy comprises the administration of non-pathogenic bacteria, **characterized in that** the composition comprises a neutrophilic granulocyte diminishing or neutrophilic granulocyte inactivating agent, which is an antibody specifically directed against neutrophilic granulocytes, an antibody specifically directed against a cytokine or chemokine attracting neutrophilic granulocytes, or a cytotoxin with anti-neutrophilic granulocyte specificity which is selected from the group comprising a cytotoxin obtainable from *Pasteurella haemolytica* culture supernatant, staphylococcal leukocidin, soluble leukocyte toxin obtainable from *Actinobacillus actinomycetemcomitans*, a leukocidin, Panton-Valentine leukocidin *of Staphylococcus aureus*, or chlodronat.

2. Composition for use according to claim 1, **characterized in that** the neutrophilic granulocyte diminishing or inactivating agent is an agent specifically causing the transient reduction in number and/or inactivation of neutrophilic granulocytes.

3. Composition for use according to claim 1 or 2, **characterized in that** the neutrophilic granulocyte diminishing or neutrophilic granulocyte inactivating agent is a neutrophilic granulocyte depleting agent.

4. Composition for use according to one of the preceding claims, **characterized in that** the bacteria comprise an expression cassette for expressing the antibody specifically directed against neutrophilic granulocytes and/or the antibody specifically directed against a cytokine or chemokine attracting neutrophilic granulocytes.

5. Composition for use according to one of the preceding claims, **characterized in that** the antibody is selected from the group comprising antibody specifically directed against at least one of the group comprising tumor necrosis factor alpha (TNFa), interleukin 8 (IL-8), epithelial-derived neutrophil attractant (ENA-78, corresponding to CXCL5), growth - related oncogene alpha (gro-α), interferon-γ-inducible-lymphocyte-attractant chemokine (monocyte chemoattractant protein 1, MCP 1), interferon gamma inducible protein (IP - 10), or monokine induced by interferon gamma (MIG), interferon gamma inducible protein (IP - 10), monokine induced by interferon gamma (MIG), formyl-MLP, anaphylatoxin C5a, anaphylatoxin C3a, prostaglandines, prostaglandine E1, prostaglandine MIP-1α (macrophage inflammatory protein 1β), prostaglandine MIP-1β (macrophage inflammatory protein 1α), RANTES/CCL5 (RANTES = regulated upon activation, normal T-cell expressed and secreted), and/or leukocyte adhesion factor LFA-1.

6. Composition for use according to one of claims 4 to 5, **characterized in that** the expression cassette contains a saccharide inducible promoter controlling the expression.

7. Composition for use according to one of the preceding claims, **characterized in that** the neutrophilic granulocyte diminishing or neutrophilic granulocyte inactivating agent is for transient inactivation or depletion of neutrophilic granulocytes.

8. Composition for use according to one of the preceding claims, **characterized in that** the antibody is for local or systemic administration.

9. Composition for use according to one of the preceding claims, **characterized in that** the bacteria comprise an expression cassette encoding the cytotoxin with anti- neutrophilic granulocyte specificity.

10. Composition for use according to claim 9, **characterized in that** the expression cassette contains a saccharide inducible promoter controlling the expression.

11. Composition for use according to claim 1, **characterized in that** the cytotoxin is that the bacteria are *E. coli* of the phylogenetic group B2.

12. Use of a neutrophilic granulocyte diminishing or neutrophilic granulocyte inactivating agent for the production of a pharmaceutical composition for use in the therapy of solid tumors, the therapy comprising the administration of bacteria, **characterized in that** the agent is selected from antibody specifically directed against neutrophilic granulocytes, an antibody specifically directed against a cytokine or chemokine attracting neutrophilic granulocytes, or chlodronat.

13. Use according to claim 12, **characterized in that** the antibody is selected from the group comprising antibody specifically directed against at least one of the group comprising tumor necrosis factor alpha (TNFa), interleukin 8 (IL-8), epithelial-derived neutrophil attractant (ENA-78, corresponding to CXCL5), growth - related oncogene alpha (gro-α), interferon-γ-inducible-lymphocyte-attractant chemokine (monocyte chemoattractant protein 1, MCP 1), interferon gamma inducible protein (IP - 10), or monokine induced by interferon gamma (MIG), interferon gamma inducible protein (IP - 10), monokine induced by interferon gamma (MIG), formyl-MLP, anaphylatoxin C5a, anaphylatoxin C3a, prostaglandines, prostaglandine E1, prostaglandine MIP-1α (macrophage inflammatory protein 1β), prostaglandine MIP-1β (macrophage inflammatory protein 1α), RANTES/CCL5 (RANTES = regulated upon activation, normal T-cell expressed and secreted), and/or leukocyte adhesion factor LFA-1.

## Patentansprüche

1. Pharmazeutische Zusammensetzung zur Verwendung in der Therapie fester Tumoren, wobei die Therapie die Verabreichung nicht-pathogener Bakterien umfasst, **dadurch gekennzeichnet, dass** die Zusammensetzung ein neutrophile Granulozyten verminderendes oder neutrophile Granulozyten inaktivierendes Agens umfasst, welches ein spezifisch gegen neutrophile Granulozyten gerichteter Antikörper ist, ein spezifisch gegen ein Zytokin oder Chemokin, das neutrophile Granulozyten anlockt, gerichteter Antikörper ist, oder ein Zytotoxin mit einer gegen neutrophile Granulozyten gerichteten Spezifität, das aus der Gruppe ausgewählt ist, die ein aus *Pasteurella haemolytica* Kulturbestand erhältliches Zytotoxin, Staphylokokken-Leukozidin, aus *Actinobacillus actinomycetemcomitans* erhältliches lösliches Leukozytentoxin, ein Leukozidin, Panton-Valentine Leukozidin von *Staphylococcus aureus* oder Clodronat umfasst.

2. Zusammensetzung zur Verwendung nach Anspruch 1, **dadurch gekennzeichnet, dass** das neutrophile Granulozyten vermindernde oder inaktivierende Agens ein Agens ist, das spezifisch die vorübergehende Verminderung der Anzahl und/oder Inaktivierung neutrophiler Granulozyten bewirkt.

3. Zusammensetzung zur Verwendung nach Anspruch 1 oder 2, **dadurch gekennzeichnet, dass** das neutrophile Granulozyten vermindernde oder neutrophile Granulozyten inaktivierende Agens ein neutrophile Granulozyten abbauendes Mittel ist.

4. Zusammensetzung zur Verwendung nach einem der voranstehenden Ansprüche, **dadurch gekennzeichnet, dass** die Bakterien eine Expressionskassette zur Expression des Antikörpers umfassen, der spezifisch gegen neutrophile Granulozyten gerichtet ist und/oder der Antikörper spezifisch gegen ein neutrophile Granulozyten anlockendes Zytokin oder Chemokin gerichtet ist.

5. Zusammensetzung zur Verwendung nach einem der voranstehenden Ansprüche, **dadurch gekennzeichnet, dass** der Antikörper aus der Gruppe ausgewählt ist, die spezifisch gegen wenigstens eines aus der Gruppe, die den Tumomekrosefaktor alpha (TNF-α), Interleukin 8 (IL-8), Epithel-abgeleiteten Neutrophilen-Lockstoff (ENA-78, entsprechend CXCL5), Wachstums-relatives Onkogen alpha (gro-α), Interferon-γ-induzierbarer Lymphozyten-Lockstoff Chemokin (Monozyten Chemolockstoff Protein 1, MCP 1), Interferongamma-induzierbares Protein (IP - 10), oder durch Interferongamma induziertes Monokin (MIG), Interferongamma-induzierbares Protein (IP - 10), durch Interferongamma induziertes Monokin (MIG), Formyl-MLP, Anaphylatoxin C5a, Anaphylatoxin C3a, Prostaglandine, Prostaglandin E1, Prostaglandin MIP-1α (Makrophagen - inflammatorisches Protein 1β), Prostaglandin MIP-1β (Makrophagen - inflammatorisches Protein 1α), RANTES/CCL5 (RANTES=reguliert bei Aktivierung, normal T-Zellen - exprimiert und sekretiert), und/oder Leukozytenadhäsionsfaktor LFA-1 gerichtete Antikörper umfasst.

6. Zusammensetzung zur Verwendung nach einem der Ansprüche 4 bis 5, **dadurch gekennzeichnet, dass** die Expressionskassette einen Saccharid - induzierbaren Promotor enthält, der die Expression kontrolliert.

7. Zusammensetzung zur Verwendung nach einem der voranstehenden Ansprüche, **dadurch gekennzeichnet, dass** das neutrophile Granulozyten vermindernde oder neutrophile Granulozyten inaktivierende Agens für die vorübergehende Inaktivierung oder den Abbau neutrophiler Granulozyten ist.

8. Zusammensetzung zur Verwendung nach einem der voranstehenden Ansprüche, **dadurch gekennzeichnet, dass** der Antikörper für die lokale oder systemische Verabreichung ist.

9. Zusammensetzung zur Verwendung nach einem der voranstehenden Ansprüche, **dadurch gekennzeichnet, dass** die Bakterien eine das Zytotoxin mit anti-neutrophile-Granulozyten-Spezifität kodierende Expressionskassette umfassen.

10. Zusammensetzung zur Verwendung nach Anspruch 9, **dadurch gekennzeichnet, dass** die Expressionskassette einen Saccharid-induzierbaren Promotor enthält, der die Expression kontrolliert.

11. Zusammensetzung zur Verwendung nach Anspruch 1, **dadurch gekennzeichnet, dass** das Zytotoxin ist, dass die Bakterien *E.coli* der phylogenetischen Gruppe B2 sind.

12. Verwendung eines neutrophile Granulozyten vermindernden oder neutrophile Granulozyten inaktivierenden Agens zur Herstellung einer pharmazeutischen Zusammensetzung zur Verwendung in der Therapie fester Tumoren, wobei die Therapie die Verabreichung von Bakterien umfasst, **dadurch gekennzeichnet, dass** das Agens ausgewählt ist unter spezifisch gegen neutrophile Granulozyten gerichteten Antikörpern und spezifisch gegen ein neutrophile Granulozyten anlockendes Zytokin oder Chemokin gerichteten Antikörpern oder Clodronat.

13. Verwendung nach Anspruch 12, **dadurch gekennzeichnet, dass** der Antikörper aus der Gruppe ausgewählt ist, die Antikörper umfasst, die spezifisch gegen wenigstens eines aus der Gruppe gerichtet sind, die den Tumornekrosefaktor alpha (TNF-α), Interleukin 8 (IL-8), Epithel-abgeleiteten neutrophilen-Lockstoff (ENA-78, entsprechend CXCL5), Wachstums-relatives Onkogen alpha (gro-α), Interferon-γ- induzierbarer Lymphozyten-Lockstoff-Chemokin (Monozyten Chemolockstoff Protein 1, MCP 1), Interferongamma-induzierbares Protein (IP - 10), oder durch Interferongamma induziertes Monokin (MIG), Interferongamma-induzierbares Protein (IP - 10), durch Interferongamma induziertes Monokin (MIG), Formyl-MLP, Anaphylatoxin C5a, Anaphylatoxin C3a, Prostaglandine, Prostaglandin E1, Prostaglandin MIP-1α (Makrophagen - inflammatorisches Protein 1β), Prostaglandin MIP-1β (Makrophagen - inflammatorisches Protein 1α), RANTES/CCL5 (RANTES=reguliert bei Aktivierung, normal T-Zellen - exprimiert und sekretiert), und/oder Leukozytenadhäsionsfaktor LFA-1 umfasst.

## Revendications

1. Composition pharmaceutique pour utilisation dans la thérapie de tumeurs solides, laquelle thérapie comprend l'administration de bactéries non pathogènes, **caractérisée en ce que** la composition comprend un agent de réduction des granulocytes neutrophiles ou d'inactivation des granulocytes neutrophiles, qui est un anticorps spécifiquement dirigé contre les granulocytes neutrophiles, un anticorps spécifiquement dirigé contre une cytokine ou une chimiokine attirant les granulocytes neutrophiles, ou une cytotoxine de spécificité anti-granulocyte neutrophile, qui est sélectionnée parmi le groupe consistant en une cytotoxine disponible à partir du surnageant de culture *Pasteurella haemolytica,* une leucocidine staphylococcique, une toxine leucocytaire soluble disponible à partir de l'*Actinobacillus actinomycetemcomitans,* une leucocidine, une leucocidine Panton-Valentine de *Staphylococcus aureus,* ou chlodronate.

2. Composition pour utilisation selon la revendication 1, **caractérisée en ce que** l'agent de réduction ou d'inactivation des granulocytes neutrophiles est un agent provoquant spécifiquement la réduction transitoire du nombre et/ou l'inactivation des granulocytes neutrophiles.

3. Composition pour utilisation selon la revendication 1 ou 2, **caractérisée en ce que** l'agent de réduction des granulocytes neutrophiles ou d'inactivation des granulocytes neutrophiles est un agent dépressif de granulocytes neutrophiles.

4. Composition pour utilisation selon l'une des revendications précédentes, **caractérisée en ce que** les bactéries comprennent une cassette d'expression permettant d'exprimer l'anticorps spécifiquement dirigé contre les granulocytes neutrophiles et/ou l'anticorps spécifiquement dirigé contre une cytokine ou une chimiokine attirant les granulocytes neutrophiles.

5. Composition pour utilisation selon l'une des revendications précédentes, **caractérisée en ce que** l'anticorps est sélectionné parmi le groupe consistant en un anticorps spécifiquement dirigé contre au moins l'un du groupe comprenant le facteur alpha de nécrose tumoral (TNF-α), l'interleukine 8 (IL-8), l'attractant neutrophilique dérivé épithélial (ENA-78, correspondant à CXCL5), un alpha oncogène lié à la croissance (gro-α), une chimiokine attractant les lymphocytes par γ interféron, (une protéine chimioattractant des monocytes 1, MCP 1), une protéine inductible par gamma interféron (IP - 10) ou une monokine induite par gamma interféron (MIG), une protéine inductible par gamma interféron (IP - 10), monokine induite par gamma interféron (MIG), formyle-MLP, une anaphylatoxine C5a, une anaphylatoxine C3a, des prostaglandines, une prostaglandine El, une prostaglandine MIP-1α (une protéine inflammatoire macrophage 1β), une prostaglandine MIP-1β (une protéine inflammatoire macrophage 1α), RANTES/CCL5 (RANTES = lymphocyte T normal régulé par activation, exprimé et sécrété), et/ou un facteur d'adhérence des leucocytes LFA-1.

6. Composition pour utilisation selon l'une des revendications 4 ou 5, **caractérisée en ce que** la cassette d'expression contient un promoteur, inductible par saccharide, de contrôle d'expression.

7. Composition pour utilisation selon l'une des revendications précédentes, **caractérisée en ce que** l'agent de réduction des granulocytes neutrophiles ou d'inactivation des granulocytes neutrophiles est destiné à l'inactivation ou la dépression transitoire des granulocytes neutrophiles.

8. Composition pour utilisation selon l'une des revendications précédentes, **caractérisée en ce que** l'anticorps est destiné à une administration locale ou systémique.

9. Composition pour utilisation selon l'une des revendications précédentes, **caractérisée en ce que** les bactéries comprennent une cassette d'expression de codage de la cytotoxine de spécificité anti-granulocyte neutrophile.

10. Composition pour utilisation selon la revendication 9, **caractérisée en ce que** la cassette d'expression contient un promoteur, inductible par saccharide, de contrôle d'expression.

11. Composition pour utilisation selon la revendication 1, **caractérisée en ce que** la cytotoxine est telle que les bactéries sont des *E. coli* du groupe phylogénétique B2.

12. Utilisation d'un agent de réduction des granulocytes neutrophiles ou d'inactivation des granulocytes neutrophiles, pour la production d'une composition pharmaceutique pour utilisation dans la thérapie de tumeurs solides, la thérapie comprenant l'administration de bactéries, **caractérisée en ce que** l'agent est sélectionné parmi un anticorps spécifiquement dirigé contre les granulocytes neutrophiles, un anticorps spécifiquement dirigé contre une cytokine ou une chimiokine attirant les granulocytes neutrophiles ou du chlodronate.

13. Utilisation selon la revendication 12, **caractérisée en ce que** l'anticorps est sélectionné parmi le groupe consistant en un anticorps spécifiquement dirigé contre au moins l'un du groupe comprenant le facteur alpha de nécrose tumoral (TNF-α), l'interleukine 8 (IL-8), l'attractant neutrophilique dérivé épithélial (ENA-78, correspondant à CXCL5), un alpha oncogène lié à la croissance (gro-α), une chimiokine attractant les lymphocytes par γ interféron (une protéine chimioattractant des monocytes 1, MCP 1), une protéine inductible par gamma interféron (IP - 10), ou une monokine induite par gamma interféron (MIG), une protéine inductible par gamma interféron (IP - 10), une monokine induite par gamma interféron (MIG), formyle-MLP, une anaphylatoxine C5a, une anaphylatoxine C3a, des prostaglandines, une prostaglandine E1, une prostaglandine MIP-1α (une protéine inflammatoire macrophage 1β), une prostaglandine MIP-1β (une protéine inflammatoire macrophage 1α), RANTES/CCL5 (RANTES = lymphocyte T normal régulé par activation, exprimé et sécrété), et/ou un facteur d'adhérence des leucocytes LFA-1.
